Europäisches Patentamt

European Patent Office

Office européen des brevets

(19)

(11) Veröffentlichungsnummer : **0 103 664**
**B1**

(12) **EUROPÄISCHE PATENTSCHRIFT**

(45) Veröffentlichungstag der Patentschrift :
**30.12.86**

(21) Anmeldenummer : **82730113.6**

(22) Anmeldetag : **25.08.82**

(51) Int. Cl.⁴ : **A 61 M  5/20**, A 61 M  5/31

(54) Vorrichtung für die Einspritzung unter Unterdruckwirkung auf die Haut.

(43) Veröffentlichungstag der Anmeldung :
**28.03.84 Patentblatt 84/13**

(45) Bekanntmachung des Hinweises auf die Patenterteilung : **30.12.86 Patentblatt 86/52**

(84) Benannte Vertragsstaaten :
**AT BE CH DE FR GB IT LI LU NL SE**

(56) Entgegenhaltungen :
**DE-A- 2 551 993**
**DE-A- 3 019 589**
**DE-C-   596 981**
**US-A- 2 743 723**
**US-A- 4 299 219**

(73) Patentinhaber : **Wagner, Wolfgang, Dr.med.**
**Exerzierstrasse 1**
**D-1000 Berlin 65 (DE)**

(72) Erfinder : **Wagner, Wolfgang, Dr.med.**
**Exerzierstrasse 1**
**D-1000 Berlin 65 (DE)**

EP 0 103 664 B1

**Beschreibung**

Die Erfindung bezieht sich auf das Gebiet der Medizintechnik, speziell auf das Gebiet der Injektionstechnik.

Bei einer Art « Sauginjektion » wird die Haut insbesondere für die Zufuhr von flüssigen Arzneien unter die Haut mittels Unterdruckes in eine innerhalb einer Saugglocke stehende Kanüle gehoben.

Einrichtungen zur Sauginjektion vermeiden die Gefahr versehentlicher Einspritzungen in die Blutbahn und die Berührung von Knochen und Nerven. Sie wurden offensichtlich bisher nur vereinzelt in der Tiermedizin eingesetzt, um Ausweichbewegungen durch Fixierung der Kanüle zu begegnen. Für die Anwendung beim Menschen insbesondere für die Selbstinjektion der Zuckerkranken erscheint die Automatisierung der Funktionsabläufe unabdingbar.

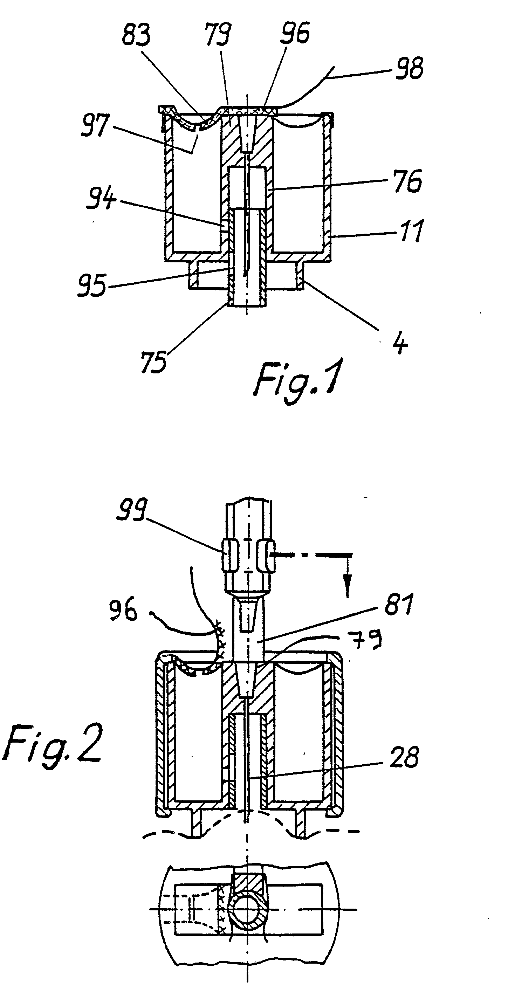

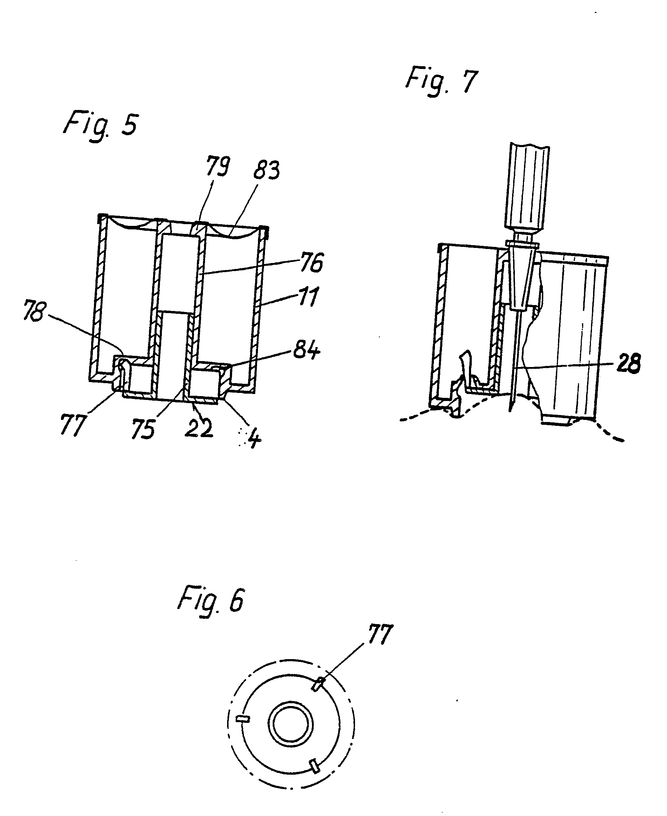

Die Technik der Einspritzung unter Unterdruckanwendung wurde bereits 1933 beschrieben (M. Demarchi 1 934 046 Verein. St. v. Amerika). Schwer transportable Einrichtungen für veterinärmedizinische Zwecke wurden von W. R. C. Geary (3 122 138 Verein. St. v. Amerika, 25.2.1964) und M. Kniazuk (3 727 614 Verein. St. v. Amerika, 17.4.1973) beschrieben. Bereits G. N. Hein (2 7-43 723, Verein. St. v. Amerika, 1.5.1956), welcher den Unterdruck durch einen Gummiball erzeugen ließ, erkannte die Schwierigkeit eines zu frühen Eindringens der Kanüle in die Haut infolge unterschiedlicher Körperoberflächenwölbung und Hautelastizität und löste die Aufgabe durch einen neben der Kanüle auf die Haut herabreichenden Sperrstift. In der DE-A-25 51 993 beschrieb ich in Figur 1b eine Erfindungslösung, bei welcher ein in einem Handgriff in der Gestalt einer Kolbenpumpe gespeicherter Unterdruck durch Eröffnen eines Ventiles vom Saugglockenrand aus in die Saugglocke hinein wirksam wird. Im Beispiel der Figur 5 der eben herangezogenen Anmeldung wird dem Trend zum Wegwerfartikel angenähert in einem die Saugglocke umgebenden Mantelraum der Sog einer äußeren Vakuumquelle zur Anhebung eines den Kanülenschaft umgebenden Kolbens benutzt, wobei der in der Vorrichtung gespeicherte Sog nachgefüllt bzw. reproduziert werden konnte.

In dem nach dem Anmeldetag der vorliegenden Anmeldung veröffentlichten Dokument DE-A-30-19 589 werden Vakuumspeicher für den Einmalgebrauch beschrieben, deren Bodenmembran oder Bodendeckel durch verschiedene Mechanismen geöffnet beziehungsweise beweglich gemacht werden, um die Haut der Unterdruckwirkung auszusetzen.

Die Abstützung von Bodenabdeckungen erwies sich als störanfällig ; außerdem mußte ein besonders nachgiebiger zentraler Bereich vorgesehen werden, welcher von der Kanüle durchdrungen werden mußte.

Hier will die Erfindung Abhilfe schaffen. Die Erfindung, wie sie in den Ansprüchen gekennzeichnet ist, löst die Aufgabe, eine Vorrichtung für die subkutane Injektion zu schaffen, dadurch, daß keine Trennwand zwischen Haut und Kanüle mehr vorgesehen ist. Es handelt sich um eine Vorrichtung für die Einspritzung unter Unterdruckwirkung auf die Haut, hauptsächlich für die Arzneiverabreichung, ins Unterhautgewebe bestehend aus einer Kammer mit einem bereits während des Herstellungsvorganges der Vorrichtung in ihr erzeugten Luftunterdruck, mit einem Deckel mit Trichter für die luftdichte Befestigung eines Behälters mit Mitteln zur Abgabe einer abgemessenen Flüssigkeitsmenge und mit einer Kanüle, befestigt an jenem Trichter und dessen Bohrung fortsetzend, wobei sich das Ende des Kanülenschaftes in eine Saugglocke erstreckt und diese Saugglocke unmittelbar an die Kammer angrenzt sowie mit dieser einen Teil von deren Bodenplatte gemeinsam hat und wobei ein Ventilmechanismus zwischen der Kammer und der Saugglocke vorhanden ist, um den Luftdruckausgleich zwischen beiden solange zu verhindern bis die Saugglocke in Druckkontakt mit der Haut ist.

Wird der Saugglockenrand gegen die Haut gepreßt, so wird in der Nähe der Kanüle und diese zunächst nach unten hin überragend ein Stift oder eine Hülse angehoben, durch den Anstau elastischer Haut sogar etwas über den Rand der Saugglocke hinweg. Dabei wird der Ventilmechanismus zwischen der sogspeichernden Kammer und der Saugglocke mit der Folge des Luftdruckausgleiches aktiviert, und der auf die Haut wirkende Unterdruck hebt diese innerhalb der Saugglocke in die Kanüle. Die Einspritzung erfolgt über eine Spritze in üblicher Weise oder über eine spezielle Dosierpumpe, welche vorzugsweise mit einer Arzneidruckflasche verbunden werden kann, Lösungen wie sie in der DE-A-25 51 991 und DE-A-25 51 992 beschrieben sind.

Die durch die Erfindung erreichten Vorteile sind im wesentlichen in einer Verbilligung der Fertigung und in einer höheren Funktionssicherheit zu sehen. Ein bereits fabrikmäßig eingeschweißter Kanülenschaft oder eine mit ihrem Ansatzstück für die Befestigung einer Spritze bereits während der Produktion fest mit der Vorrichtung verbundene Kanüle vereinfacht die Handhabung gerade für ängstliche und ungeschickte Kranke, da der Topf der Vorrichtung besser angefaßt werden kann und die Kanüle als verletzendes Werkzeug gar nicht sichtbar wird. Die Mitnahme des Injektionszubehörs zum Gebrauch unterwegs wird durch Fortfall eines besonderen Hilfswerkzeuges zur Einführung einer Kanüle erleichtert. Voraussetzung ist eine gewisse Reibung zwischen Spritzenkolben und Zylinder, um eine vorzeitige Entleerung der Spritze im Augenblick der Anhebung der Haut zu verhindern oder der Einsatz einer Kolbensperre, etwa einer Klemme unmittelbar über dem Spritzenzylinder auf dem Spritzenkolben. Diese Klemme muß dann entweder vor der Einspritzung wieder entfernt werden oder sie verschiebt sich federnd gleitend während der Senkung des Spritzenkolbens durch Fingerdruck auf denselben. Bei Glasspritzen sind solche Klemmen häufig bereits in den Zylinderdeckel eingebaut. Werden Spritzen ohne solche Abstimmung der Kolben-Zylinder-Reibungsverhältnisse verwendet, so ist eine Hilfsvorrichtung erforderlich, welche erlaubt, nach Abziehen einer klebenden Folie über dem zentralen konischen Trichter für die Befestigung der Spritze im Deckel dieselbe ohne Sichtkontrolle der Vorrichtung im Zustand der Kanülenabdichtung durch Hautansaugung anzunähern ; andernfalls würde man einen Hauptvorteil der Sauginjektion opfern, nämlich den, fast beliebige Hautstellen zur Injektion auswählen zu können und durch Wechsel der Injektionsstellen die Haut zu schonen. Sofern keine Wundnarben vorliegen, ist selbst die Einspritzung über der Halsschlagader völlig unbedenklich. Eine klebende Folie kann kennzeichnenderweise auch so beschaffen sein, daß sie sich über eine Öffnung in dem Deckel der sogspeichernden Kammer hinweg fortsetzt, so daß weiterer Zug an einer freihängenden Lasche der klebenden Folie durch Freilegung jener Öffnung ein Eindringen der Außenluft und damit eine Wiederbelüftung auch im Saugglockenbereich bewirkt, so daß die Haut sich aus der Kanüle zurückzieht und die Vorrichtung nach Aufhebung der Haftung an der Haut mit der Spritze weggenommen werden kann.

Die Aufgabe, ein vorzeitiges Eindringen der Kanülenspitze in die Haut noch vor der Vakuumeinwirkung auf dieselbe zu verhindern, wird erfindungsgemäß in einem Beispiel dadurch gelöst, daß die Kanüle von einer die Ebene des Saugglockenrandes überragenden Hülse umgeben ist, welche innerhalb eines Binnenzylinders der Kammer nach oben hin verschieblich ist. Zweckmäßigerweise dient diese Hülse dazu, einen Ventilmechanismus zwischen der Kammer und dem Binnenzylinder zu eröffnen zur Einleitung des Luftdruckausgleiches zur Saugglocke hin. Bei dem Mechanismus kann es sich um Öffnungen handeln, welche durch diese Verschiebung der Teile gegeneinander zur Deckung gebracht werden. In einem anderen Beispiel trägt die Hülse einen Bodenring, von dem wenigstens ein Arm mit seinem Ende nach einer Sollbruchstelle in der Bodenplatte der Kammer sich erstreckt, um diese Sollbruchstelle bei Anhebung der Hülse durch den Hautandruck zu eröffnen. In wieder einem anderen Beispiel wird eine Ventilöffnung in der Bodenplatte von einem Folienring unter luftdichter Verklebung einer ringförmigen Zone um die Ventilöffnung herum verdeckt, wobei die Folie sich hutartig in einem Spalt zwischen Binnenzylinder und Hülse nach oben hin fortsetzt, um das obere Ende der Hülse wenigstens teilweise zu überspannen oder an dieser befestigt zu sein. Wird die Hülse angehoben, so wird der Folienrand von der Ventilöffnung in der Bodenplatte weggezogen und so der Luftdruckausgleich eingeleitet. In einer Variation des Beispiels liegt ein Reißfaden innerhalb der die Ventilöffnung abdichtenden Folie, welcher sich zum oberen Ende der Hülse erstreckt und bei deren Anhebung das Wegziehen oder Zerreißen der dichtenden Folie bewirkt. Vorzug im Hinblick auf Funktion und Herstellungskosten verdient aber eine Lösung, bei welcher exzentrisch neben dem Kanülenschaft in der Kammer ein Rohrstutzen angeordnet ist, welcher durch die Bodenplatte hindurch in die Saugglocke offen ist und von einem Ventilstift ausgefüllt wird, welcher etwa auf der Ebene des Saugglockenrades nach unten hin endet. Dieser Ventilstift ist so beschaffen, daß er den Rohrstutzen zunächst gegen die Saugglocke abdichtet, nach Anhebung aber den Luftabfluß in die Kammer freigibt. Bei einer solchen Ventilkonstruktion kann der Binnenzylinder entfallen und die Kanüle sich durch die gesamte Kammer hindurch erstrecken bis in die Saugglocke hinein. Das Kanülenansatzstück — sofern nicht ein Trichter gleicher Funktion als Teil des Deckels mit einem Kanülenschaft verbunden vorgesehen ist — ist dicht mit dem Deckel der Kammer verschweißt ; ebenso ist — zweckmäßigerweise innerhalb eines ganz kurzen Rohrstutzens — der Kanülenschaft innerhalb der Bodenplatte dicht verschweißt oder befestigt. Der Ventilstift weist günstigerweise eine ringförmige Randleiste dicht unter dem oberen Ende des Rohrstutzens und eine weitere tiefer gelegene eventuell mit Längsdurchbrüchen für den Luftdurchtritt auf. Eine Wachs- oder Siegelschicht zwischen Ventilstift und Rohrstutzen gewährleistet den dauerhaften Luftabschluß. Wird durch Druck der Haut auf den Ventilstift dieser nur eine ganz geringe Strecke angehoben, so bricht ruckartig diese Siegelschicht zwischen Ventilstift und Rohrstutzen durch, die obere Randleiste verläßt den Rohrstutzen, der jetzt einer weiteren Verschiebung unter dem Sog einfluß von der Hauptkammer her auf die sich hebende Haut keinen Widerstand mehr bietet. Diese Verkürzung des Arbeitshubes des Ventilmechanismus erleichtert die Verpackung — vorzugsweise bei geldrollenartiger Stappelung — und bietet vorallem Gewähr dafür, daß der Sogspeicher erst dann eröffnet wird, wenn der Saugglockenrand allseitig und vollständig von Haut verschlossen ist.

In einer Variation zu diesem Beispiel ist wiederum eine Art Binnenzylinder vorgesehen, jetzt aber als Einsenkung von oben. um einen Spritzenzylinder unter Verkürzung des Kanülenschaftes dichter an die Einspritzstelle heranzubringen. Es wird so die Dosiergenauigkeit erhöht und der Arzneiverlust verringert, welcher sich aus der zurückbleibenden Restmenge innerhalb des Kanülenschaftes ergibt. In einem solchen Falle entfernt sich der Rohrstutzen und damit auch der Ventilstift von der Kanüle. Von dem Ventilstift erstreckt sich in diesem Falle ein Ring zum Zentrum hin, welcher die Kanüle umgibt. Der Ventilstift benötigt eine Drehsicherung. In der dargestellten Variation endet der Ventilstift nach oben hin in eine Art Kanüle, welche zum Luftdruckausgleich eine Stopfenmembran im Rohrstutzen durchbricht.

Eine gegen die sogspeichernde Kammer gedichtete Rohrverbindung zwischen Deckel und Bodenplatte, welche die Saugglocke mit der Außenluft verbindet, kann sowohl zur Wiederbelüftung über Lüftung einer Deckfolie als auch zur Prüfung herangezogen werden, ob nach dichtendem Hautkontakt der Ventilmechanismus eröffnet wurde, um den richtigen Zeitpunkt zur Einspritzung zu wählen. Die dargestellte Säckchenkanüle, welche sich unter Sogeinfluß zusammenziehen soll, dient dabei der erfindungsgemäßen Demonstration.

Eine Membranabdeckung über einer Deckelöffnung oder eine membranöse Verdünnung des Deckels der Kammer dient mit Hilfe von deren Einziehung unter Vakuumeinfluß der Kontrolle, ob die Vorrichtung sich noch in funktionsgerechtem Zustand befindet.

Als Fertigungsmaterial können beispielsweise weniger gasdurchlässige Chargen von Polystyrol für die Vorrichtung gewählt werden, für eine Hülse mit Bodenplatte und einem als Ventilstift dienendem Arm beispielsweise Polyvinylchlorid. Die Versiegelung des Rohrstutzens mit dem Ventilstift erfolgt beispielsweise unter Ultraschall oder durch Diathermie oder über die Hitzeeinwirkung bei der Aufschweißung des Deckels auf die Kammer innerhalb einer Unterdruckkammer. Eine Vorrichtung mit einem Rohrstutzen als Teil des Ventilmechanismus kann auch zuerst mit Deckel versehen werden, wonach in der Unterdruckkammer die Abdichtung des Ventilstiftes innerhalb des Rohrstutzens erfolgt.

Im folgenden wird die Erfindung anhand von Zeichnungen, welche verschiedenen Ausführungsbeispielen entsprechen, näher erläutert. Es zeigt

Figur 1 einen Längsschnitt durch ein Ausführungsbeispiel im Zustande vor Gebrauch, bei welchem der Kanülenschaft fester mit in den Deckel der Kammer einbezogener Bestandteil ist.

Figur 2 einen Längsschnitt durch ein Ausführungsbeispiel nach Figur 1 während des Gebrauchs mit einer Hilfsvorrichtung zur Annäherung der Spritze. Unmittelbar darunter befindet sich eine ausschnittsweise Draufsicht.

Figur 3 einen Längsschnitt durch das bevorzugte Ausführungsbeispiel mit einem Ventilstift neben der Kanüle.

Figur 4 einen Längsschnitt durch eine Variation des Ausführungsbeispieles der Figur 3 während des Gebrauchs.

Figur 5 einen Längsschnitt durch ein Ausführungsbeispiel vor Gebrauch, welches eine verschiebliche Hülse mit Bodenring und Arm als Elemente des Ventilmechanismus unter einer Sollbruchstelle der Bodenplatte.

Figur 6 eine schematische Draufsicht eines Ausführungsbeispieles wie in Figur 5 und Figur 7 im Längsschnitt gezeigt.

Figur 7 einen Längsschnitt durch ein Ausführungsbeispiel wie in Figur 5 während des Gebrauchs.

Figur 8 eine Ansicht, teilweise im Längsschnitt von einer Hilfsvorrichtung zur Einführung einer Spritze mit Kanüle.

Figur 9 einen Längsschnitt vom unteren Teil eines Ausführungsbeispieles zur Darstellung eines Ventilmechanismus.

Figur 10 einen Längsschnitt vom unteren Teil eines Ausführungsbeispieles zur Darstellung eines anderen Ventilmechanismus.

Figur 11 einen schematischen Längsschnitt in der Vergrößerung 2 : 1 eines Ausführungsbeispiels ähnlich wie in Figur 1, wobei Einrichtungen zur Wiederbelüftung und Kuppelung zur Darstellung kommen. Figur 12 zeigt eine Variante der Fig. 3.

In Figur 1 wird — ausführlicher beschrieben — eine Vorrichtung gezeigt, wie sie aussieht, wenn sie gerade der Verpackung entnommen wurde. Sie besteht aus einem Außenzylinder (11) mit einem zentralen Trichter (79), welcher sich nach unten zu in einen festeingebauten Kanülenschaft hinein fortsetzt. Ein Binnenzylinder (76), welcher sich nach unten in die Saugglocke (4) fortsetzt, umschließt den größeren Teil des Kanülenschaftes. Eine Hülse (75) ist innerhalb des Binnenzylinders verschieblich und überragt den Saugglockenrand unten. Eine schlitzförmige seitliche Öffnung (95) liegt unterhalb der seitlichen Öffnung (94) des Binnenzylinders. Eine adhäsive Masse dichtet den Raum zwischen dieser seitlichen Öffnung (94) und der Wand der Hülse (75). Die Ringmembran (83) wird durch den Luftunterdruck ins Innere der Außenkammer hineingezogen und wird von einer Klebefolie so überspannt, daß diese mit ihrer klebenden Unterseite ein Loch (97) in der Ringmembran (83) und den Trichter (79) abdichtet. Die Folie hat ein freies Ende (98).

Figur 2 zeigt ein Ausführungsbeispiel wie dasjenige in Figur 1, bei welchem eine mit ihrer Klammer (99) eine Spritze haltende Halbrinne (81) so auf einer die Injektionsvorrichtung umschließenden Mantelhülse befestigt ist, daß die gebrauchsfertig vorbereitete Spritze mit ihrem Ansatzkonus direkt über die Trichteröffnung (79) zu liegen kommt. Der dargestellte Zustand, in welchen — dargestellt durch eine gestrichelte Linie — die Haut in die Saugglocke hineingezogen ist und die Kanülenspitze durchdrungen hat, ist ein Ergebnis folgender Handhabung. Die Vorrichtung wurde mit dem Saugglockenrand gegen die Haut gedrückt, wobei die Hülse 75 innerhalb des Binnenzylinders angehoben wurde und die Öffnungen des Ventilmechanismus zur Deckung kamen. Die dadurch bewirkte Ausgleichung des Luftdruckes zwischen der sogspeichernden Kammer und der von der Haut abgedichteten Saugglocke bewirkte deren Anhebung. Jetzt wurde die Folie (96) an ihrem freien Ende (98) so weit nach links abgezogen, daß die Trichteröffnung freigelegt wurde. Es kann jetzt die Spritze längs der Halbrinne 81 nach unten verschoben werden, bis ihr Ansatzkonus fest innerhalb des Trichters liegt. Die Einspritzung selbst wird in üblicherweise dadurch vollzogen, daß Druck auf den Spritzenkolben über dessen Stempel ausgeübt wird. Ein zusätzlicher Zug am freien Ende (98) der Klebefolie (96) legt das Loch (97) in der Ringmembran (83) frei, so daß Außenluft über den Binnenzylinder in die Saugglocke eindringen kann. Die Haut fällt innerhalb der Saugglocke von selbst zurück und verläßt die Kanüle 28 und die Saugglocke. In einer — nicht abgebildeten — vereinfachten Variation erstreckt sich die Klebefolie (96) nicht über den Trichter. In diesem Falle wird eine Spritze mit ihrem Ansatzkonus bereits vor Auslösung des Ventilmechanismus zum Luftdruckausgleich dicht im Trichter des Deckels der Vorrichtung befestigt.

Die Vorrichtung kann auch mit dem Ansatzstutzen einer Dosiervorrichtung verbunden werden, wie sie in der Offenlegungsschrift 25 5-1 991 beschrieben wurde.

Direkt unter dem Längsschnitt der Figur 2 ist eine teilweise Draufsicht dargestellt.

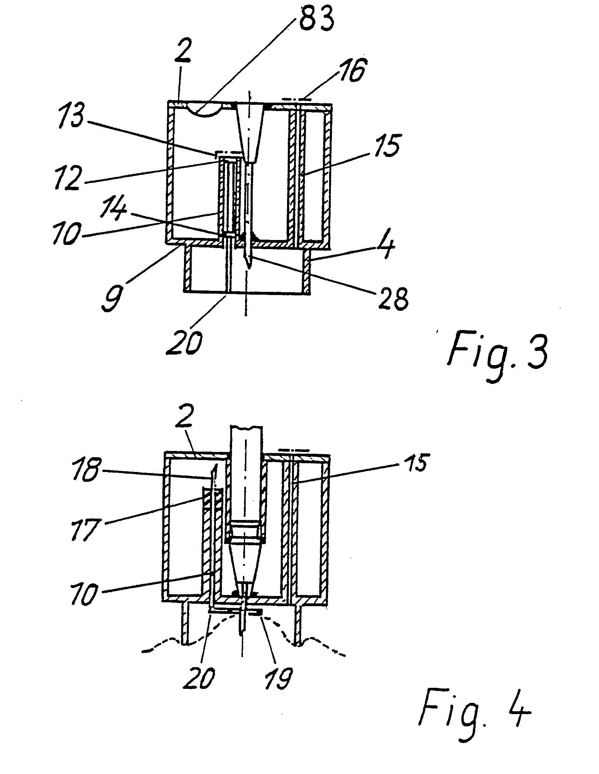

Figur 3 zeigt in einem Längsschnitt das bevorzugte Ausführungsbeispiel, bei welchem eine Kanüle mit ihrem Verbindungsstück zwischen Kanülenschaft (28) und Spritze dicht im Zentrum eines festen Deckels (2) verschweißt ist. Gegen die darunter liegende Saugglocke (4) zeigt die Kammer für die Vakuumspeicherung als Trennwand eine Bodenplatte (9), welche vom Ende des Kanülenschaftes durchdrungen wird bei Dichtung desselben innerhalb der Bodenplatte. Exzentrisch findet sich ein Rohrstutzen (10) in der Bodenplatte, welcher in die Kammer hineinragt und einem Ventilstift (20) als Aufnahme dient. Der Ventilstift berührt mit einer oberen ringförmigen Randleiste (12) die Innenwand im oberen Ende des Rohrstutzens, wobei die Randleiste zur Innenwand des Rohrstutzens hin durch eine Wachsschicht (13) gedichtet ist ; eine untere ringförmige Randleiste (14) des Ventilstiftes mit Längsunterbrechungen für den Luftdurchtritt liegt nahe dem unteren Ende des Rohrstutzens und befördert die Lagestabilisierung des Ventilstiftes, welcher in Höhe des Saugglockenrandes endet. Zwischen dem Deckel (2) und der Bodenplatte (9) in die Saugglocke (4) hinein sich öffnend findet sich eine feine Röhre (15), welche vor Gebrauch unter Verwendung von Spritzen durch ein Dichtungspflaster (16) verschlossen ist, dessen Entfernung nach der Einspritzung der Wiederbelüftung der Saugglocke dienen kann. Eine Dünnstelle im Deckel (2) oder eine Öffnung dort, welche von der Membran (83) verschlossen wird, läßt an deren Einziehung in den Kammerraum erkennen, daß dort Unterdruck vorhanden ist, was eine Voraussetzung für die Verwendungsfähigkeit der Vorrichtung ist.

Wird die Vorrichtung auf die Haut aufgesetzt, so drängt die innerhalb des Saugglockenrandes aufgestaute Haut den Ventilstift nach oben, wobei das Wachssiegel zerbricht und die obere Randleiste des Ventilstiftes den Rohrstutzen verläßt, so daß Luft aus der Saugglocke in die Kammer eindringt. Dadurch wird die Haut in die Kanüle gehoben und kann die Einspritzung — wie oben beschrieben — vollzogen werden.

Figur 4 zeigt im Längsschnitt eine Variation zum Ausführungsbeispiel der Figur 3, bei welcher der Rohrstutzen (10) oben von einem Dichtpfropfen (17) aus Gummi verschlossen ist. Der Ventilstift (20) trägt eine nach unten hin zur Seite offene Kanüle (18) an seinem oberen Ende und ist durch eine Abplattung gegenüber einer entsprechend vorspringenden Leiste im Rohrstutzen drehgesichert. An seinem unteren Ende trägt er den zentral gelegenen Ring (19). Im dargestellten Stadium einer unter Vakuumeinfluß von der Kammer her hochgezogenen — gestrichelt wiedergegebenen — Haut wurde der Ventilstift bereits zuvor durch den Druck der Haut auf den Ring (19) angehoben. Die Kanüle (18) des Ventilstiftes hat dabei das verdünnte Zentrum des Dichtpfropfens durchbrochen und den Druckausgleich zwischen Kammer und Saugglocke bewirkt.

Figur 5 illustriert im Längsschnitt eine Abwandlung der Vorrichtung, wie sie in den Figuren 1 und 2 dargestellt wurde. Nach dieser Verkörperung der Erfindung weist der Binnenzylinder (76) oben einen zentralen Trichter (79) auf, um das Verbindungsstück zwischen Schaft und dosierendem Arzneibehälter einer Kanüle — die hier nicht gezeigt wird — aufzunehmen. Der Rand der Saugglocke (4) ist von dem die Saugglocke umgebenden Außenzylinder (11) besonders abgesetzt. Die Bodenplatte als Grenze zwischen Saugglocke und Kammer weist eine Wandverdünnung (84) auf, welche sich nischenartig in die Seitenwandung der Saugglocke fortsetzt und in welche drei federnd nach außen gebogene Arme (77) eingerastet sind, welche vom Bodenring (22) an der Hülse (75) ausgehen. Die drei Arme sind auch in den Figuren 6 und 7 dargestellt und gleichmässig auf den Kreisumfang verteilt. Wird Druck auf den Bodenring (22) ausgeübt, so können die zugespitzten Enden (78) der Arme (77) die Wandverdünnung (84) leicht durchbrechen. Die Wandverdünnung kann auch durch Folien ersetzt sein, welche entsprechende Fensteröffnungen in der Bodenplatte verschliessen. In evakuiertem Zustand wird die Kammer von der Ringmembran (83) verschlossen.

Figur 6 zeigt in einer schematischen Draufsicht die Enden der Arme (77) in ihrer Rastposition.

Figur 7 zeigt einen Axialschnitt mit teilweiser Seitenansicht einer Vorrichtung wie in Figur 5, aber in Verbindung mit einer aufgesteckten Kanüle (28), welche ihrerseits mit einer Spritze verbunden ist. Man erkennt wie das Ende eines Armes die Wandverdünnung der Bodenplatte durchbrochen hat und die Haut — gestrichelt dargestellt — in die Kanülenspitze hineingehoben wurde, nachdem die Luft des Binnenzylinder, der eine Innenkammer (26) bildet, infolge des Druckgefälles zur Außenkammer (25) hin teilweise abströmen konnte.

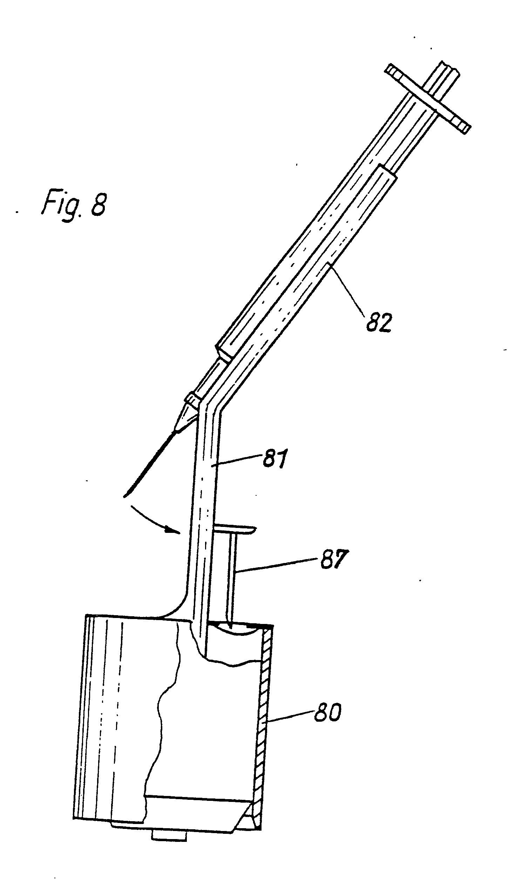

Figur 8 zeigt eine Hilfsvorrichtung, um eine Kanüle in die Vorrichtung entsprechend den Figuren 5 bis 10 einzuführen. Die Hilfsvorrichtung besteht aus einem zylindrischen Topf (80), welcher nach unten hin offen ist und über eine Injektionsvorrichtung gestülpt wurde. Die Halbrinne für die Führung der Spritze weist einen senkrechten Teil (81) auf und endet in einem schrägen (82). Die üblicherweise gefüllte und mit Kanüle versehene Spritze wird zunächst in den oberen schrägen Teil der Halbrinne eingelegt und während einer Verschiebung nach unten in die Senkrechte gekippt, was eine Einführung der Kanüle in den Trichter der Injektionsvorrichtung erlaubt, ohne die Sterilität durch Berührung zu gefährden. Die Senkung des Dornes (87) bewirkt Zerstörung der Membran.

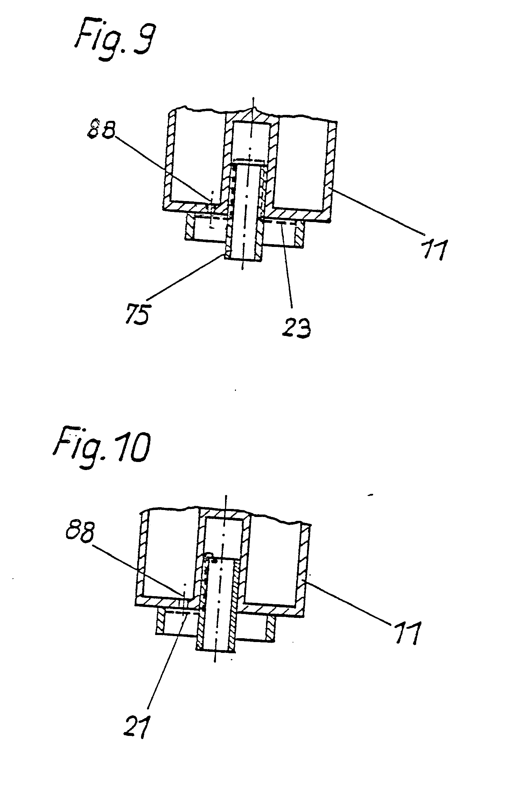

Die Figur 9 zeigt im Längsschnitt den unteren Teil einer Vorrichtung entsprechend der in den Figuren 1 und 2 dargestellten unter Hinweglassung der Kanüle. Der Ventilmechanismus zwischen Kammer und Saugglocke ist abgewandelt. Die sogspeichernde Kammer weist zur Saugglocke hin die Öffnung (88) auf, welche durch den ringförmigen Rand einer um die Öffnung durch Klebemasse gedichteten hutförmigen Membran (23) verschlossen ist. Die hutförmige Membran setzt sich bis über das obere Ende der Hülse (75) fort, welche unten den Saugglockenrand überragt. Das über dem Hülsenende liegende Zentrum der Membran (23) läßt entweder Raum für den Durchtritt der Kanüle oder wird von dieser durchstochen.

Wird die Hülse durch Druck auf die Haut angehoben, so wird der Rand der hutartigen Membran von der Öffnung weggezogen, und der Druckausgleich zwischen Kammer und Saugglocke findet statt.

Figur 10 gibt eine der Figur 9 entsprechende Darstellung, jedoch findet sich lediglich über der Öffnung (88) eine adhäsive Membran. Diese Membran enthält einen Reißfaden (21) eingeschweißt, dessen geknotetes Ende in einem Schlitz am oberen Ende der Hülse befestigt ist. Wird die Hülse angehoben, so bewirkt Zug am Reißfaden die Beseitigung der Membran über der Ventilöffnung und damit den Luftdruckausgleich.

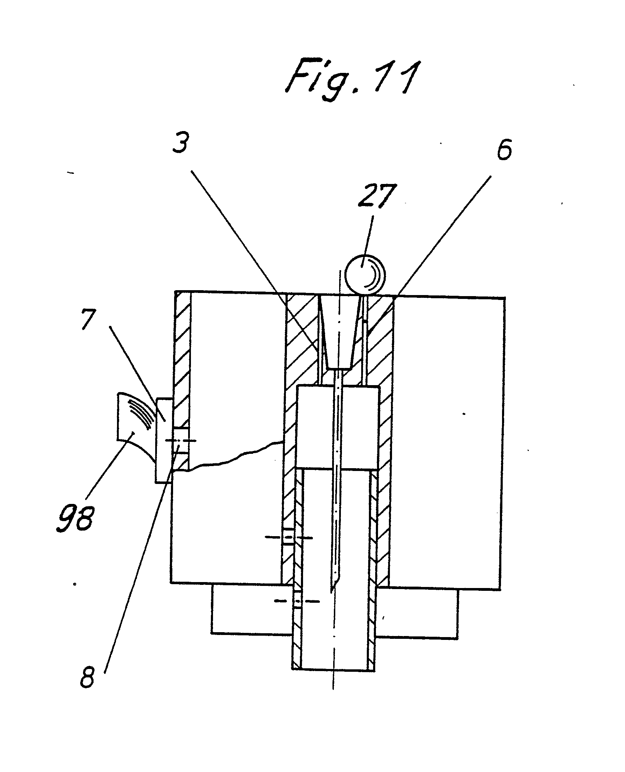

Die Figur 11 zeigt im Längsschnitt schematisch und im Maßstabe 2 : 1 vergrößert eine Vorrichtung wie diejenige in Figur 1. Zusätzlich wird eine Bohrung (3) aus der Schräge des Trichters senkrecht in den Binnenzylinder geführt. Solange der Trichter vom Ansatzkonus einer Spritze verschlossen ist, kann keine Luft entweichen bei dichtem Hautkontakt nach Eröffnung des Ventilmechanismus. Wird der Ansatzkonus der Spritze nach der Injektion abgezogen, so findet durch die Bohrung (3) eine Wiederbelüftung der Saugglocke statt. Ein zusätzlicher Kanal (6) kann nützlich beim Gebrauch in Verbindung mit einem automatischen Injektor sein. In einem solchen Falle kann auch einmal eine Bohrung (8) in der Außenwand der Kammer von einem Klebeband (7) verschlossen sein, dessen Entfernung die Wiederbelüftung einleitet. Der Sack (27) einer in den Kanal (6) gedichtet eingeführten Säckchenkanüle Muß — bei Lageverhältnissen, wie abgebildet — vom Ansatzkonus der Spritze etwas seitlich abgedrängt werden ; er kollabiert, sobald der Ventilmechanismus bestimmungsgemäß betätigt wurde.

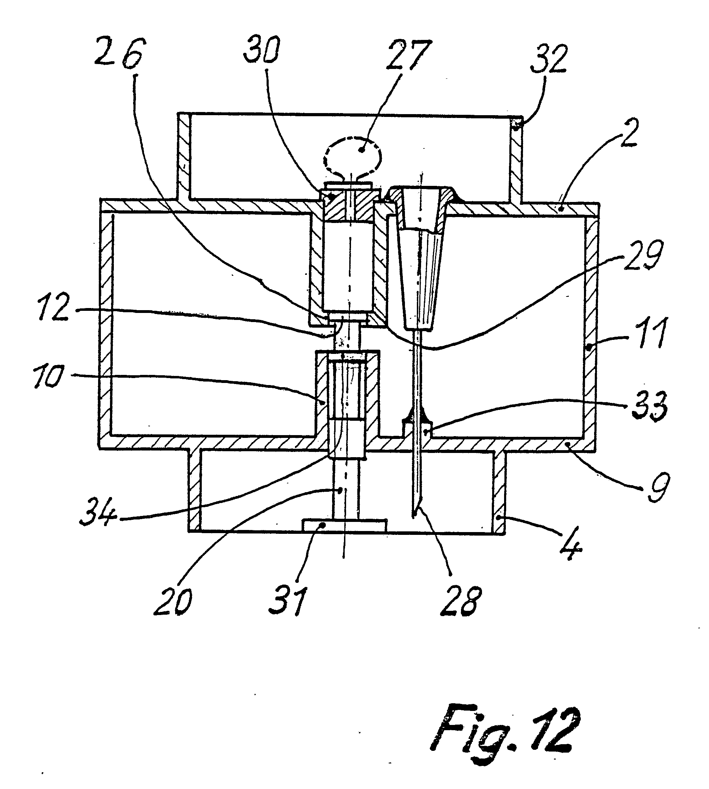

Die Figur 12 bringt im Längsschnitt bei einer Vergrößerung im Maßstabe 2 : 1 eine Abwandlung des Ausführungsbeispieles der Figur 3, wobei Rohrstutzen und Kanüle zur deutlicheren Darstellung zusätzlich etwas auseinandergezogen sind. Die Kammer (11), welche als Vakuumspeicher dient, ist etwas breiter und dafür gedrungener gestaltet, um eine Verkürzung des Kanülenschaftes (28) zu erzielen. Zentral liegt in diesem Beispiel der Rohrstutzen (10), welcher in seinem oberen Ende vom unteren Ende der kolbenartigen Randleiste (12) des Ventilstiftes (20) verschlossen wird, dessen unteres Ende die Andruckplatte (31) trägt. Die kolbenartige Randleiste (34) setzt sich nach oben hin einen freien Spaltraum in der Kammer überbrückend in das untere Ende der Rohrmuffe fort, welche den Kanal (26) umschließt, und dichtet diesen ab. Unmittelbar oberhalb des Endes des Ventilstiftes erweitert sich der Kanal (26) in der Ausbuchtung (29). Den Abschluß des Kanals nach oben bildet die dichtende Stopfenplatte (30) mit einer Bohrung, welche in ein Gummisäckchen (27) endet. Das Verbindungsstück einer Kanüle ist im Deckel (2) unmittelbar neben dessen Ausstülpung für den Kanal (6) dicht verschweißt. Innerhalb der kurzen Rohrmuffe (33) ist der Kanülenschaft in der Bodenplatte (9) dicht verschweißt, wobei die Kanülenspitze in die Saugglocke (4) hineinragt. Der Deckel (2) trägt noch eine Randtülle (32), in welche der Zylinder einer anderen Saugglocke eingeschoben werden kann zur besseren Stapelung verschiedener Vorrichtungen übereinander.

Zum Gebrauch wird eine gefüllte Injektionsspritze in das Verbindungsstück der Kanüle dichtend eingeführt. Die Saugglocke wird unter Zug an der Kammer von der Randtülle der darunterliegenden Vorrichtung abgezogen. Eine Sterilität gewährleistende — nicht dargestellte — Verpackungsbanderole war schon zuvor entfernt worden. Nun wird der Rand der Saugglocke (4) gegen die Haut gepreßt, welche infolge ihrer Elastizität sich unter der Andruckplatte (31) des Ventilstiftes (20) anstaut, bis unter Anhebung desselben die Ventilverbindung zwischen der kolbenartigen Randleiste (12) und dem Rohrstutzen (10) sowie der Rohrmuffe des Kanals (26) gesprengt wird und infolge Luftdruckausgleiches sowohl die Haut in die Kanüle gehoben als auch das Gummisäckchen (27) zusammengezogen wird. Die Herabsetzung der wandberührenden Fläche durch Verdoppelung der kolbenartigen Randleiste bei Hinzufügen einer die Führung des Ventilstiftes unterstützenden Ringleiste (33) desselben im unteren Teil des Rohrstutzens (10) setzt auch die Reibungswiderstände herab.

Die beschriebenen Erfindungselemente können auch in anderen Injektoren eingesetzt, kombiniert und variiert werden, ohne damit aufzuhören Bestandteil dieser Erfindung zu sein. So kann ein von einer Hülse ausgehender nach oben abgewinkelter einzelner Arm als Ventilstift in einem Rohrstutzen innerhalb der Kammer enden. Die Kammer kann quadratische oder polygonale Wandbegrenzung aufweisen zur Erhöhung der Griffigkeit, zur Verhinderung des Wegrollens und zur Verminderung des Verpackungstotraumes. Solche Abweichungen, wie sie in den Beispielen nur unvollständig aufgezählt werden konnten, befinden sich noch innerhalb der Reichweite der Erfindung. Insbesondere ist es nicht von Belang, welche Elemente — und ob überhaupt irgendwelche — ins Zentrum der Saugglocke verlegt werden. Auch die Saugglocke selbst kann von der Kreisform abweichen, wie Versuche ergeben haben.

**Patentansprüche**

1. Vorrichtung für die Einspritzung unter Unterdruckwirkung auf die Haut, hauptsächlich für die Arzneiverabreichung ins Unterhautgewebe, bestehend aus einer Kammer (11) mit einem bereits während des Herstellungsvorganges der Vorrichtung in ihr erzeugten Luftunterdruck, mit einem Deckel (2) mit Trichter (79) für die luftdichte Befestigung eines Behälters mit Mitteln zur Abgabe einer abgemessenen Flüssigkeitsmenge und mit einer Kanüle (28), befestigt an jenem Trichter (79) und dessen Bohrung fortsetzend, wobei sich das Ende des Kanülenschaftes in eine Saugglocke (4) erstreckt und diese Saugglocke (4) unmittelbar an die Kammer (11) angrenzt sowie mit dieser einen Teil von deren Bodenplatte (9) gemeinsam hat und wobei ein Ventilmechanismus (94, 95 ; 10, 12, 14, 20 ; 75, 77, 78, 84 ; 75, 88, 23 ; 75, 88, 21) zwischen der Kammer und der Saugglocke vorhanden ist, um den Luftdruckausgleich zwischen beiden solange zu verhindern bis die Saugglocke in Druckkontakt mit der Haut ist.

2. Vorrichtung nach Patentanspruch 1, bei dem ein membranöser Teil (83) des Deckels (2) vorgesehen ist, um zu prüfen, ob der Unterdruck innerhalb jener Kammer (11) vorhanden ist.

3. Vorrichtung nach Patentanspruch 1, bei der anstelle des Trichters (79) das Verbindungsstück zwischen dem Kanülenschaft (28) und dem Behälter zur Abgabe einer abgemessenen Flüssigkeitsmenge fester Bestandteile des Deckels (2) ist.

4. Vorrichtung nach Patentanspruch 1, bei der die Kammer (11) als größere und unterdruckspeichernde Außenkammer von einer Innenkammer durch den Binnenzylinder (76) abgegrenzt wird, wobei innerhalb letzterem eine Hülse (75) um einen Teil des Kanülenschaftes (28) so verschieblich angeordnet ist, daß die Verschiebung dieser Hülse (75) den Ventilmechanismus (94, 95 ; 75, 77, 78, 84 ; 75, 88, 23 ; 75, 88, 21) eröffnet.

5. Vorrichtung nach Patentanspruch 4, bei der der Ventilmechanismus aus mindestens einer Öffnung (94) in der Wandung des Binnenzylinders (76) besteht, welche mindestens einer Öffnung (95) in der Hülse (75) entspricht und wobei diese sich gegenseitig entsprechenden Öffnungen durch Verschiebung der Hülse (75) unter dem Einfluß des Hautandruckes miteinander zur Deckung gebracht werden.

6. Vorrichtung nach Patentanspruch 4, bei der die Hülse (75) einen Bodenring (22) aufweist mit wenigstens einem stiftartigen Arm (77) und die Bodenplatte (9) mit wenigstens einer Sollbruchstelle (84) versehen ist, wobei das Ende (78) des Armes (77) dazu angepaßt ist, diese Sollbruchstelle (84) zu durchbrechen, wenn die Hülse (75) durch den Hautandruck gegen die Kanüle (28) angehoben wird.

7. Vorrichtung nach Patentanspruch 4, bei der dieser Ventilmechanismus aus wenigstens einer Öffnung (88) zwischen der Kammer (11) und der Saugglocke (4) besteht, welche luftdicht durch eine Folie (23) verschlossen ist, wobei letztere so mit der Hülse (75) verbunden ist, daß die Folie (23) von der Öffnung (88) soweit beseitigt wird, daß der Luftdruckausgleich stattfindet, wenn die Hülse (75) unter dem Hautandruck aufwärts verschoben wird.

8. Vorrichtung nach Patentanspruch 1, bei der eine Kanüle (28) fest und luftdicht innerhalb des Deckels (2) befestigt ist und sich durch das Innere der Kammer (11) durch deren Bodenplatte (9) in die Saugglocke (4) hinein erstreckt und dabei gegen die Bodenplatte (9) gedichtet ist.

9. Vorrichtung nach Patentanspruch 1, bei welcher der Ventilmechanismus aus einem Rohrstutzen (10) besteht, welcher nahe dem Kanülenschaft (28) mit seinem unteren Teil in die Bodenplatte (9) gedichtet eingefügt ist und von einem Ventilstift (20) verschlossen wird.

10. Vorrichtung nach Patentanspruch 9, bei welcher der Ventilstift (20) mindestens eine ringförmige Randleiste (12, 14 ; 33) aufweist, davon eine vor der Auslösung des Ventilmechanismus innerhalb des oberen Endes des Rohrstutzens (10).

11. Vorrichtung nach Patentanspruch 1, bei der als Dichtungsmittel zwischen dem unbewegten Teil (76 ; 10) und dem bewegten Teil (75 ; 20) des Ventilmechanismus eine dichtende Masse vorhanden ist, deren Anhäufung verteilt wird, während durch den Hautandruck der Ventilmechanismus betätigt wird.

12. Vorrichtung nach Patentanspruch 1, bei der sich zur Kammer (11) gedichtet ein Kanal (15) durch den Deckel (2) bis zur Bodenplatte (9) erstreckt bei Öffnung zur Saugglocke (4) hin, um die Öffnung des Ventilmechanismus festzustellen oder zur Wiederbelüftung der Saugglocke (4).

**Claims**

1. A device for injection effected by negative pressure against the skin, particularly for the administering of drugs into the subcutaneous tissue, comprising a chamber (11) in which reduced pressure is produced already during the manufacturing process, with a covering lid (2) with a funnel (79) for the air-tightened insertion of a container with means for applying measured amounts of fluid and with a cannula (28) fastened to said funnel (79) and spatially continuing said funnel, the cover end of the cannula shaft extending into a suction cup (4) and said suction cup (4) immediately touching said chamber (11) and having a base plate (9) together with it, and a valve mechanism (94, 95 ; 10, 12, 14, 20 ; 75, 77, 78, 84 ; 75, 88, 23 ; 88, 21) being present between said chamber and said suction cup in order to prevent the compensation in the difference of the air pressure between said chamber and said suction cup, until said suction cup is in pressure contact with the skin.

2. A device according to claim 1, wherein a membranaceous portion (83) of said covering lid (2) is provided for checking the influence of negative pressure inside of said chamber (11).

3. A device according to claim 1, wherein instead of said funnel (79) the connection piece between the cannula shaft (28) and the container for applying a measured amount of flulid is a firm component of said covering lid (2).

4. A device according to claim 1, wherein said chamber (11) is marked off as a biger and negative pressure storing outer chamber from an inner chamber by the inner cylinder (76) inside of the latter a sleeve (75) being arranged in a manner as to open the valve mechanism (94, 95 ; 10, 12, 14, 20 ; 75, 77, 78, 84 ; 75, 88, 23 ; 88, 21) by the shifting of said sleeve.

5. A device according to claim 4, wherein said valve mechanism consists of at least one opening (94) in the wall of said inner cylinder (76), corresponding to at least one opening (95) in said sleeve (75), said corresponding openings being in a coinciding position by the shifting of said sleeve (75) under the influence of the pressing-on of the skin.

6. A device according to claim 4, wherein said sleeve (75) has a bottom ring (22) with at least one pin-like projection (77) and wherein the bottom plate (9) is provided with at least one breaking seal (84) while said sleeve (75) is lifted toward said cannula (28) by the pressing-on of the skin.

7. A device according to claim 4, wherein said valve mechanism consists of at least one opening (88) between said chamber (11) and said suction cup (4) which is air-tightened closed by a foil (23), the latter being connected with said sleeve (75) such a manner that the foil is removed from said opening (88) as far as to complete the compensation of air pressure, while said sleeve (75) is shifted upwardly by the pressing-on of the skin.

8. A device according to claim 1, wherein a cannula (28) is air-tightly fastened inside of said covering lid (2) and extends through the inside of said chamber (11) through its bottom plate (9), to which it is sealed, into said suction cup (4).

9. A device according to claim 1, wherein said valve mechanism consists of a pipe socket (10) which is air-tightly inserted onto sair bottom plate (9) with its lower portion near to said cannula shaft (28) being closed by a valve pin (20).

10. A device according to claim 9, wherein said valve pin (20) has at least one ring shaped rim ledge (12, 14 ; 33), one of which inside of the upper end of said pipe socket (10) before the release of said valve mechanism.

11. A device according to claim 1, wherein a adhesive mass is present as sealing means between the immovable part (76 ; 10) and the movable part (75 ; 20) of the valve mechanism, the accumulation thereof being distributed during the activation of the valve mechanism by the pressing-on of the skin.

12. A device according to claim 1, wherein a channel (15) extends through the covering lid (2) to the bottom plate (9) which is tightened toward the chamber (11) while opened toward the suction cup (4), so that the opening of the valve mechanism may be checked or the suction cup (4) may be reventilated.

**Revendications**

1. Dispositif pour l'injection sous l'effet de dépression vers la peau surtout pour l'administration d'un médicament sous le tissu subcutané constitué d'une chambre (11) avec une pression atmosphérique diminuée produit déjà pendant le procédé de la production du dispositif avec un couvercle (2) avec un entonnoir (79) pour une consolidation hermétique d'un récipient avec des moyens pour la délivrance d'une quantité de liquide et avec une canule (28), consolidée sur cet entonnoir (79) et prolongeant son forage, la fin de la tige de la canule s'étendant dans la cloche d'aspiration (4) et cette cloche d'aspiration avoisinant la chambre (11) et ayant commune avec cela une partie de la lame basale (9) et existant un mécanisme de ventilation (94, 95 ; 10, 12, 14, 20 ; 75, 77, 78, 84 ; 75, 88, 23 ; 75, 88, 21) entre la chambre et la cloche d'aspiration pour l'équilibration de la pression atmosphérique entre l'une et l'autre jusqu'à ce que la cloche d'aspiration est en contact de compression avec la peau.

2. Dispositif selon la revendication 1, auprès de lui une partie membraneuse (83) du couvercle (2) est prévue pour prouver si la dépression existe à l'intérieur de cette chambre (11).

3. Dispositif selon la revendication 1, auprès de lui, au lieu de l'entonnoir (79) il y a la pièce de jonction entre la tige de la cannule (28) et le récipient pour la remise d'une quantité mesurée qui est une partie stable du couvercle (2).

4. Dispositif selon la revendication 1, auprès de lui la chambre (11) en tant que chambre extérieure majeure et accumulante de dépression, séparée d'une chambre intérieure par un cylindre intérieur (76) dans lequel est arrangée une douille (75) autour d'une partie de la tige de la canule (28), coulissant afin que le déplacement de cette douille (75) ouvre le mécanisme de ventilation (94, 95 ; 75, 77, 78, 84 ; 75, 88, 23 ; 75, 88, 21).

5. Dispositif selon la revendication 4, auprès de lui le mécanisme de ventilation consiste au moins d'un orifice (94) dans la cloison du cylindre intérieur (76) qui corresponde au moins d'un orifice (93) dans la douille (75) et par laquelle ces orifices correspondent l'un avec l'autre font coïncidence par le déplacement de la douille (75) sous l'effet de pression de la peau.

6. Dispositif selon la revendication 4, auprès de lui la douille (75) présente un anneau basal (22) avec du moins un bras (77) d'une manière de stylet et la lame basale (9) sont dotés avec du moins un endroit (84) prévu, le bout (78) du bras (77) étant adapté pour enfoncer cet endroit (84), quand la douille (75) est soulevée par la pression sur la peau vers la canule (28).

7. Dispositif selon la revendication 4, auprès de lui ce mécanisme de ventilation ci-dessus consiste d'au moins un orifice (88) entre la chambre (11) et la cloche d'aspiration (4) et qui est isolé hermétiquement par une feuille (23), la dernière étant conjugée avec la douille (75) afin que la feuille (26) soit mise de côté de l'orifice à ce point que l'égalisation de la pression atmosphérique a lieu, quand la douille (75) est soulevée par la pression sur la peau.

8. Dispositif selon la revendication 1, auprès de lui une canule (28) est consolidée fermement et hermétiquement à l'intérieur d'un couvercle (2) et pénétrant à travers l'intérieur de la chambre (11) par la lame basale (9) dans la cloche d'aspiration (4) en même temps étant rendue étanche dans la lame basale (9).

9. Dispositif selon la revendication 1, auprès de lui le mécanisme de ventilation consiste d'un raccord de tuyau (10) qui est inséré hermétiquement près de la tige de la canule (28) avec sa partie inférieure dans la lame basale et est fermé par un poussoir de ventilation (20).

10. Dispositif selon la revendication 9, auprès de lui le poussoir de ventilation (20) présente au moins une bordure annulaire (12, 14, 33) de laquelle une est située devant le déclenchement du mécanisme de ventilation en sein du bout supérieur du raccord de tuyau (10).

11. Dispositif selon la revendication 1, auprès de lui en tant que moyen pour étancher l'espace entre la partie immobile (76, 10) et la partie mise en mouvement (75, 20) du mécanisme de ventilation, il y a une masse étanche dont l'accumulation est distribuée, pendant que le mécanisme de ventilation est actionné par la pression de la peau.

12. Dispositif selon la revendication 1, auprès de lui un canal (15) s'étend étanchement vers la chambre (11) au travers du couvercle (21) jusqu'à la lame basale (9) tout en étant ouvert vers la cloche d'aspiration (4) pour constater l'ouverture du mécanisme de ventilation ou pour la reventilation de la cloche d'aspiration (4).

9

*Fig.1*

*Fig.2*

Fig. 3

Fig. 4

Fig. 7

Fig. 5

Fig. 6

Fig. 8

Fig. 9

88

75

23

11

Fig. 10

88

21

11

# Fig. 11

Fig.12